# EUROPEAN PATENT SPECIFICATION

(11) **EP 4 463 867 B1**
(45) Date of publication and mention of the grant of the patent: **20.05.2026**
(21) Application number: 23700713.3
(22) Date of filing: 11.01.2023
(51) Int. Cl.: G16B 20/30, G16B 30/10, G16B 40/20

(54) **SYNTHETIC PROMOTERS GENERATED BASED ON GENOMIC DNA SEQUENCES**
BASIEREND AUF GENOMISCHEN DNA-SEQUENZEN ERZEUGTE SYNTHETISCHE PROMOTOREN
PROMOTEURS SYNTHÉTIQUES GÉNÉRÉS À PARTIR DE SÉQUENCES D'ADN GÉNOMIQUE

(30) Priority: 11.01.2022 EP 22150941
(43) Date of publication of application: 20.11.2024
(73) Proprietor: Danmarks Tekniske Universitet, 2800 Kongens Lyngby (DK)
(72) Inventor: SIMONSEN, Henrik Toft, 2800 Kongens Lyngby (DK); WORKMAN, Christopher, 2800 Kongens Lyngby (DK); ROTHSCHILD-MANCINELLI, Kyle, 2800 Kongens Lyngby (DK)
(74) Representative: Høiberg P/S
(86) International application number: PCT/EP2023/050499
(87) International publication number: WO 2023/135151

(56) References cited:
- TEAM DTU: "iGEM 2019 project webpage", 24 February 2020 (2020-02-24), XP055931209, Retrieved from the Internet <URL:https://2019.igem.org/Team:DTU-Denmark> [retrieved on 20220614]
- GILMAN JAMES ET AL: "Rapid, Heuristic Discovery and Design of Promoter Collections in Non-Model Microbes for Industrial Applications", vol. 8, no. 5, 17 April 2019 (2019-04-17), Washington DC ,USA, pages 1175 - 1186, XP055929744, ISSN: 2161-5063, Retrieved from the Internet <URL:http://pubs.acs.org/doi/pdf/10.1021/acssynbio.9b00061> [retrieved on 20220610], DOI: 10.1021/acssynbio.9b00061
- RENDSVIG JAKOB K. H. ET AL: "Bidirectional histone-gene promoters in Aspergillus: characterization and application for multi-gene expression", vol. 6, no. 1, 9 December 2019 (2019-12-09), XP055930049, Retrieved from the Internet <URL:http://link.springer.com/article/10.1186/s40694-019-0088-3/fulltext.html> [retrieved on 20220610], DOI: 10.1186/s40694-019-0088-3

## Description

The present disclosure relates to methods for generation of synthetic promoters and/or terminators, and sequences obtained by such a method.

### Background of invention

Synthetic biology is an emerging interdisciplinary field that involves the application of engineering principles to biology. It aims to use artificial biological pathways, organisms and devices to study natural systems and to provide new solutions for pressing medical, industrial and environmental problems. In recent years, significant advances have been made in DNA synthesis capabilities for a wide range of biological circuits. However, the problem of restricted scalability of the process remains and the current challenges in the design, construction and validation of synthetic biological systems need to be addressed in order to achieve the scalable higher-complexity biological circuits. Present methods for the design of promoters and terminators are typically based on testing of a large number of random sequences, or just minimizing existing DNA sequences. These approaches rely on molecular and experimental data, and commonly require a prior knowledge on expression and functionality, in order to obtain a strong and useful regulatory DNA sequence. The project from the Technical University of Denmark for the 2019 "International Genetically Engineered Machine" (iGEM) competition (https://2019.igem.org/Team:DTU-Denmark) discloses a method for generating synthetic promoter sequences by iteratively optimizing a consensus sequence. This sequence is obtained from a Hidden Markov model applied to an alignment of homologous putative promoter sequences. The project does not relate to establishing a statistical model for activity regulating sequence features.

### Summary of the inventon

The invention is set out in the appended claims

### Summary of related disclosure

The present inventors have realized how synthetic promoters and terminators can be generated in a highly scalable way, wherein said promoters and terminators not only work under many different conditions but for example also have the same relative gene expression from each other, irrespective of the scale.

Typically, the synthetic promoter and/or terminator are intended for expressing a protein of interest in a cell. For example, wherein said synthetic promoter and/or terminator are to be used for controlling the level of expression of said protein of interest in the cell.

The present disclosure therefore, in a first aspect, relates to a computer-implemented method for generating a synthetic promoter and/or terminator for expressing a protein of interest in a cell, the method comprising:
- obtaining gene sequences, such as functional sequences and/or coding sequences, of genomes of multiple closely related species;
- identifying at least one homolog group of said genomes, each homolog group comprising multiple gene sequences that are homologs;
- identifying gene statistics for each gene sequence of the homolog groups, wherein the gene statistics are based on performance parameters and are a prediction of the strength of promoters or terminators of each respective gene sequence, preferably wherein the performance parameters are selected from the group consisting of: transcription rate, preferably based on tRNA adaptation index and/or number of effective codons; transcription profiling, preferably based on RNA sequencing data; and protein quantification, preferably measured by mass spectrometry;
- identifying, for each homolog group, a set of functional DNA contexts comprising, for each gene sequence of respective homolog group:
   ∘ in the case of generation of a synthetic promoter: an upstream DNA sequence, said upstream DNA sequence being upstream of the start codon of the gene sequence, in the respective genome, and having a length greater than the expected length of the promoter in said given genome, or
   ∘ in the case of generation of a synthetic terminator: a downstream DNA sequence, said downstream DNA sequence being downstream of the stop codon of the gene sequence, in the respective genome, and having a length greater than the expected length of the terminator in said given genome,
- building, for each homolog group, a multiple sequence alignment (MSA), each MSA consisting of the set of functional DNA contexts of said homolog group,
- iteratively fitting, for each MSA, a hidden markov model (HMM), wherein the fitting is repeated until convergence and/or until a predetermined number of iterations have been carried out, thereby obtaining a starting consensus sequence for each MSA,
- identifying multiple model features of the HMM, the model features being indicative of regions of high conservation in said starting consensus sequences, and generating a max-score matrix (FMS) comprising the maximum scores for each model feature for each HMM,
- forming a statistical model, such as PLSR, for modeling gene statistics as a function of FMS;
- identifying activity regulating features, by calculating a correlation coefficient between parameters of the statistical model and features scores of the FMS, and recognizing said activity regulating features as model features of which said correlation coefficient is above a predetermined threshold, such as 0.5,
- optimizing, for each HMM, the starting consensus sequence, by, for each activity regulating feature, calculating the gene statistics upon inserting/replacing the activity regulating feature into the starting consensus sequence or a partially optimized consensus sequence, and
   ∘ where said insertion/replacement leads to an improvement in the gene statistics, such as an increase by a predetermined threshold, accepting said insertion/replacement,
   ∘ otherwise said insertion/replacement is discarded;
thereby generating a synthetic promoter and/or terminator for expressing a protein of interest in a cell.

Typically, the method of the present disclosure has an intended technical use for expressing a protein of interest in a cell. The intended use may for example comprise:
i. providing a cell comprising a gene sequence encoding the protein of interest,
ii. inserting a synthetic promoter, and/or a synthetic terminator, obtained by the method according to the present disclosure, i.e. into said gene sequence,
iii. incubating the cell in a medium,
whereby the cell expresses the protein of interest.

In a preferred embodiment of the present disclosure, the synthetic promoter is inserted upstream of said (target) gene sequence, and/or the synthetic terminator is inserted downstream of said (target) gene sequence.

Alternatively or additionally, the intended technical use may comprise or consist of determining the strength of the synthetic promoter and/or terminator relative to the strength of the native promoter or of the native terminator. This intended technical use may comprise:
i. synthesizing an artificial sequence comprising: the synthetic promoter and a target gene sequence downstream of the synthetic promoter; and/or the synthetic terminator and a target gene sequence upstream of the synthetic terminator;
ii. introducing said artificial sequence in a target genome;
iii. measuring transcription levels from the target gene sequence; and
iv. comparing the transcription levels measured in step iii) with the transcription levels obtained from the same target coding sequence with the native promoter or with the native terminator.

The obtained gene sequences are typically measured sequences of related species existing in the physical reality. Thus, the computer-implemented method may favourable interact with the external physical reality.

In an embodiment of the present disclosure, the method further comprises a step (preferably after the step of optimizing, for each HMM, the starting consensus sequence): of synthesizing an artificial sequence comprising: the synthetic promoter and a target gene sequence downstream of the synthetic promoter; and/or the synthetic terminator and a target gene sequence upstream of the synthetic terminator.

In specific examples of the present disclosure, the artificial sequence is introduced in a target genome. Thus, the present disclosure may comprise a further step of introducing the artificial sequence in a target genome.

Preferably, the method comprises a step of measuring transcription levels from the target gene sequence.

Preferably, the method comprises a step of comparing the measured transcription levels with the transcription levels obtained from the same target coding sequence with the native promoter or with the native terminator;
thereby determining the strength of the synthetic promoter and/or terminator relative to the strength of the native promoter or of the native terminator.

Thus, the present disclosure may further comprise one or more of the following steps (preferably after the step of optimizing, for each HMM, the starting consensus sequence):
i. synthesizing an artificial sequence comprising: the synthetic promoter and a target gene sequence downstream of the synthetic promoter; and/or the synthetic terminator and a target gene sequence upstream of the synthetic terminator; and/or
ii. introducing said artificial sequence in a target genome; and/or
iii. measuring transcription levels from the target gene sequence; and/or
iv. comparing the transcription levels measured in step iii) with the transcription levels obtained from the same target coding sequence with the native promoter or with the native terminator.

In addition to being highly scalable, the method, or parts thereof, also does not rely on molecular and experimental data, but rather automation making the method highly cost-efficient, fast and versatile. The method may be modified according to specific needs of a promoter and/or terminator, for example the promoter and/or terminator may be generated to have a specific strength, for example as compared to a native promoter and/or terminator.

The gene sequences may for example be obtained from publicly accessible online databases, such as National Center for Biotechnology Information (NCBI) and/or joint genome institute (JGI). Alternatively or additionally, the gene sequences may be sequenced de novo. Typically, at least 10 closely related species are obtained.

The species may for example be selected from any of the following genera:
*Aspergillus, Saccharomyces, Kluyveromyces, Komagataella, Barnettozyma, Cyberlindnera, Phaffomyces, Physcomitrium, Starmera, Wickerhamomyces, Yarrowia* or *Trichoderma.* Preferably, the species are selected from the same genus, and thus the species, and the genomes thereof, can be considered closely related.

The obtained gene sequences may for example be genome assemblies, and may be annotated or unannotated. Thus, in specific embodiments of the present disclosure, the method may comprise a step of annotation comprising annotation of protein encoding genes.

The method typically comprises identifying at least one homolog group of the genomes of multiple closely related species, e.g. species within the same genera. Each homolog group comprises multiple gene sequences that are homologs, for example at least 2 gene sequences, more preferably at least 10 gene sequences, even more preferably at least 30 gene sequences, yet even more preferably at least 50 gene sequences, once yet even more preferably at least 100 gene sequences, most preferably at least 500 gene sequences.

The method further typically comprises identification of gene statistics, for each gene sequence of the homolog groups. The gene statistics are preferably based on performance parameters such as transcription rate, transcription profiling and/or protein quantification.

For each homolog group, a set of functional DNA contexts is preferably identified, said functional DNA context being either an upstream DNA sequence or a downstream DNA sequence with respect to the gene sequence, for the generation of a synthetic promoter or a terminator respectively. Each set of functional DNA contexts is preferably used to perform a multiple sequence alignment (MSA), thereby a set of MSAs is obtained. Each of said MSAs is preferably used to fit a hidden markov model (HMM).

Each HMM typically consists of a set of symbols on top of an underlying, but unobservable, Markov process. Each state in the process contains probabilities for emitting each symbol when the process enters that state. The sequence of states cannot be observed directly, only the sequence of emitted symbols. An advantage with the HMM is that it takes into account not only point mutations but also the possibility of insertions and deletions.

Regions of each model may contain one or more stretches of high conservation. High conservation is typically indicated by high information content (IC) in the match states of the model. The high IC regions are preferably used to identify, for each HMM, a number of model features that in turn is used to model the gene statistics as a function of the features.

The correlation between the parameters of the statistical model and the features scores of the FMS is preferably used to determine activity regulating features of each model, which may be considered to be features that modify the activity of the promoter and/or terminator. The method preferably involves testing the resulting gene statistics upon insertion of the identified activity regulating features in an iterative process that is based on the consensus sequence.

In a further aspect, the present disclosure relates to a statistical model for generation of synthetic promotes and/or terminators. The statistical model may for example be a partial least square regression model, and may further have been generated as disclosed elsewhere herein, for example based on gene statistics of and model features of one or more consensus sequences.

In yet a further aspect, the present disclosure relates to a data processing system for generating synthetic promoters and/or terminators. The system preferably comprises an input device, a central processing unit, a memory, and an output device, wherein said data processing system has stored therein data representing sequences of instructions which when executed cause a method for generating a synthetic promoter and/or terminator to be performed, the memory preferably further comprises a statistical model, such as a partial least square regression model.

In yet a further aspect, the present disclosure relates to a computer software product containing sequences of instructions for generation of synthetic promoters and/or terminators. The computer software product may for example comprise instructions that, when executed, cause a method for generating a synthetic promoter and/or terminator to be performed.

In even yet a further aspect, the present disclosure relates to an integrated circuit product containing sequences of instructions for generation of synthetic promoters and/or terminators. The computer software product may for example comprise instructions that, when executed, cause a method for generating a synthetic promoter and/or terminator to be performed.

In a further aspect, the present disclosure relates to a data processing system for identifying synthetic promoter and/or terminator sequences. The system may for example comprise an input device, a central processing unit, a memory, and an output device, wherein said data processing system has stored therein data representing sequences of instructions which when executed, cause a method for generating a synthetic promoter and/or terminator to be performed as disclosed herein. In a further aspect, the present disclosure relates to a method for expressing a protein of interest in a cell using synthetic promoter. Said method comprising:
I. providing a cell comprising a gene sequence encoding the protein of interest,
II. inserting a synthetic promoter, and/or a synthetic terminator, such as obtained by a method for generating a synthetic promoter and/or terminator to be performed as disclosed elsewhere herein, such as into said gene sequence,
III. incubating the cell in a medium,
whereby the cell expresses the protein of interest.

In a preferred embodiment of the present disclosure, the synthetic promoter is inserted upstream of said (target) gene sequence, and/or the synthetic terminator is inserted downstream of said (target) gene sequence. Preferably in such a way as to control the expression of the (target) gene sequence.

### Description of drawings

**Fig. 1** shows a flow chart illustrating a method for generating a synthetic promoter and/or terminator according to an embodiment of the present disclosure.
**Fig. 2** shows results of protein production measurements of optimized promoters in comparison with the native promoters of two separate organisms according to an embodiment of the presently disclosed method.
**Fig. 3** shows the promoter activity measurements for 14 synthetic phase 1 design (black) and native (white) pairs with percent increases indicated for each pair.
**Fig. 4** shows predicted improvements between synthetic phase 2 and phase 1 designs for 241 selected *Komagatella* orthogroup promoter models.

### Detailed description of the invention

As used herein, the term "promoter" or "promoter sequence" refers to a regulatory DNA sequence which when ligated to a nucleotide sequence of interest is capable of controlling the transcription of the nucleotide sequence of interest into RNA. A promoter is typically, though not necessarily, located 5' (i.e., upstream) of a nucleotide sequence of interest and provides a site for specific binding by RNA polymerase and other transcription factors for initiation of transcription.

As used herein, the term "terminator" or "terminator sequence" refers to a DNA sequence at the end of a transcriptional unit which signals termination of transcription, initiated from a promoter. Preferably, a transcription terminator sequence furthermore comprises sequences which cause polyadenylation of the transcript. A transcription terminator may, for example, comprise one or more polyadenylation signal sequences, one or more polyadenylation attachment sequences, and downstream sequence of various lengths which causes termination of transcription. It has to be understood that also sequences downstream of sequences coding for the 3'-untranslated region of an expressed RNA transcript may be part of a transcription terminator although the sequence itself is not expressed as part of the RNA transcript.

Furthermore, a transcription terminator may comprise additional sequences, which may influence its functionality, such as 3'-untranslated sequences (i.e. sequences of a gene following the stop-codon of the coding sequence). Transcription termination may involve various mechanisms including but not limited to induced dissociation of RNA polymerase II from their DNA template.

As used herein, the term "homolog" is used to indicate polynucleotide sequences, possessing a high degree of sequence relatedness due to shared ancestry. Such relatedness may be quantified by determining the degree of identity and/or similarity between the two sequences as hereinbefore defined. Falling within this generic term are also the terms "ortholog" and "paralog". Two segments of DNA can have shared ancestry because of three phenomena: either a speciation event (orthologs), or a duplication event (paralogs), or else a horizontal (or lateral) gene transfer event (xenologs).

As used herein, the term "multiple sequence alignment" or "MSA" refers to the sequences of multiple homologs of a starting gene that are aligned using an algorithm (e.g., ClustalW, MUSCLE, etc.).

As used herein, the term "Hidden Markov Model" or "HMM" refers to a statistical Markov model in which the system being modeled is assumed to be a Markov process (i.e., a stochastic model describing a sequence of possible events in which the probability of each event depends only on the state attained in the previous event) with unobservable states. HMM are fit for each MSA in an iterative procedure. During each iteration, the current HMM model is used to create a new MSA that is then used to fit a new HMM model. In each iteration, the original set of sequences are used in the new alignment (i.e. the sequences are not trimmed) but the model is allowed to vary in the number of match states. Convergence is tested by extracting the consensus sequence from each model and comparing it to consensus sequences from previous iterations. If the same consensus sequence is observed, the model optimization process is deemed to have converged. The most probable sequence from the HMM can be considered a consensus sequence.

As used herein, the term "consensus sequence" refers to the sequence formed from the most frequently occurring amino acids (or nucleotides) in a family of related sequences.

In a first aspect, the present disclosure relates to a method for generating a synthetic promoter and/or terminator, involving obtaining gene sequences, building sequence models and optimizing DNA designs based on activity regulating features and gene statistics.

The method typically comprises obtaining gene sequences; identifying homolog groups and gene statistics of said gene sequences; identifying, for each homolog group, a set of functional DNA contexts; building, for each homolog group, a multiple sequence alignment (MSA) fitted to a hidden markov model (HMM); identifying multiple model features of the HMM and generating a max-score matrix (FMS); calculating a correlation coefficient between parameters of the statistical model and features scores of the FMS; and optimizing, for each HMM, the starting consensus sequence.

In one embodiment of the present disclosure, the step of obtaining the gene sequences comprises species identification, data acquisition, data harmonization and/or genome annotation.

The method for generating a synthetic promoter and/or terminator may target the generation of synthetic promoters and/or terminators for a specific organism, for example wherein the synthetic promoters and/or terminators are intended for use in a protein production process using said organism. The identification of species may therefore comprise identifying a number of closely related species, related to said organism. This may comprise identification of a number of species within the same genus or taxonomical family as said organism.

Various sources for obtaining gene sequences, e.g. assembled genomes, exist. The sources may comprise for example the National Center for Biotechnology Information (NCBI) and/or Joint Genome Institute (JGI) databases. Often, multiple assemblies exist for a given species and in these cases a specific gene sequence must be selected, which is most often identified as the "reference" genome.

Data harmonization may comprise harmonization of the format and/or at least parts of the contents of the obtained gene sequences. The gene sequences may consist of or be based on genome assembly projects, which could be organized and distributed in a number of ways. Such assemblies are available in a number of stages of completeness and either unannotated or annotated, i.e. gene features identified and mapped to this assembly. Genome assemblies available from public data resources, e.g. NCBI and JGI, are typically already partly harmonized although they may require a few additional harmonization steps. In particular, it is a preference that the file containing the genomic DNA, referred to here as "genomic.fna", is available as a multi Fasta file. Annotated gene sequences, such as genomes, will typically have a number of additional files but, in general, minimally include an annotation file in the General Feature Format (GFF), ideally version 3 format, referred to here as "genomic.gff". If a gene sequence, such as a genome, is annotated, it will in general minimally include the identification of protein coding sequences, known as CDS.

In general it is preferred that the location (e.g. path and file name) of the genomic.fna and possible genomic.gff files is registered in a database or table that describes the taxonomic data (Family, Genus, Species, taxon_id) and, importantly, the genetic code (gencode) used to translate the nuclear or plastid encoded protein genes. Prokaryotes will typically have one gencode, apart from plants and algae eukaryotes which typically have 2 (nuclear and mitochondrial), whereas eukaryotic algae and plants will typically have 3 (nuclear, mitochondrial and chloroplast). For this approach, it is a preference that at least the nuclear gencode is provided.

For instances wherein the gene sequences are unannotated, an annotation tool may be used in order to identify protein coding sequences, such as YGAP or FGENSH+. Further, it is a preference that the annotation of the gene sequences comprises annotation of tRNA genes. In fact, in a preferred embodiment of the present disclosure, the step of obtaining a gene sequence comprises annotation of tRNA genes and/or other protein encoding genes.

While some genome annotation pipelines annotate tRNA and rRNAs others do not. Therefore, it is generally a preference that, in embodiments of the present disclosure comprising a step of annotation, e.g. as part of a step of obtaining a gene sequence, all tRNA genes of the obtained gene sequences e.g. of genomic.fna, are annotated, such as by using tRNAscan-SE. The output from the program may be filtered to generate a tRNA Gene Copy Number (GCN) table for each annotated genome. The GCN table, which may be used for calculating a tRNA adaptation index (tAl), typically contains each possible anticodon and the number of tRNA genes that perfectly match this anticodon.

In an embodiment of the present disclosure, gene sequences of preferably at least 10 closely related species are obtained, such as at least 20 or 30 or even 40 closely related species, more preferably at least 50 closely related species, such as at least 60, 70, 80 or even 90 closely related species, even more preferably at least 100 closely related species.

In a further embodiment of the present disclosure, the genomes and/or gene sequences are obtained from databases, such as databases comprising genome assemblies, and/or sequenced de novo. Examples of databases include, but are not limited to, NCBI and JGI.

In one embodiment of the present disclosure, the gene sequences are annotated. In some instances, the gene sequences obtained from databases are fully or partially annotated. In some instances, gene sequences obtained from databases include a file containing the genomic DNA as a multi Fasta file. In some instances, gene sequences obtained from databases include a file in the General Feature Format (GFF), preferably version 3 format. In some instances, gene sequences obtained from databases include the identification of protein coding sequences, known as CDS. In some instances, protein coding genes may be annotated using a number of tools including but not limited to YGAP and FGENSH+. In some instances, tRNA genes are annotated using tRNAscan-SE.

In an embodiment of the present disclosure, the method comprises a step of identifying at least one homolog group, each homolog group comprising multiple gene sequences that are homologs. Said step may comprise analyzing each gene sequence for the presence of homologs, e.g. orthologs and/or paralogs. Each homolog group preferably comprises at least one part of multiple gene sequences.

In an embodiment of the present disclosure, the coding sequences of each gene sequence, e.g. of each genome, may be analyzed for homologs. This may comprise or consist of performing an analysis of the protein sequences with tools such as KOG, OthroMCL, OrthoFinder, etc. The output of this analysis may be a set of homolog groups (HGs), where preferably each HG comprises or consists of a set of genes across the different closely related species, such as if the genomes of said species comprises one or more homologs in this group. To avoid HGs that are only defined for 1 or very few species, a minimum threshold is preferably applied in order to generate a list of HGs with sequences from at least *n* or more species. In practice, if *n*=5, the number of OGs is similar to the number of coding genes in any one species, e.g. ~6000 in yeasts. Consequently, in a preferred embodiment of the present disclosure, the homologs of the homolog groups are identified in at least n species, wherein n is 5 or higher, more preferably 7 or higher, yet more preferably 10 or higher.

In one embodiment of the present disclosure, the closely related species are of the same genus and/or related genera within a family.

In a further embodiment of the present disclosure, the method comprises a step of identifying gene statistics for each gene sequence of the homolog groups, for example wherein the gene statistics are based on performance parameters and are a prediction of the strength of promoters or terminators of each respective gene sequence.

It has been observed that the extent of codon optimization in a gene's coding sequence (CDS) is highly correlated to the transcription rate of the mRNA of that gene. In this way, codon optimization may be used as a measure of promoter activity and mRNA levels in addition to its ability to for example predict protein translation rates and subsequent protein levels.

In a preferred embodiment of the present disclosure, the step of identifying gene statistics may comprise or consist of calculating and/or measuring one or more performance parameters, for example parameters that are predictors of the strength of promoters and/or terminators of each respective gene sequence. Said performance parameters may comprise or consist of transcription rate, transcription profiling, and/or protein quantification. The calculation and/or measurement of said performance parameters may therefore involve the use of measurement equipment and/or algorithm for identifying said performance parameters.

In an embodiment of the present disclosure, the transcription rate is, or is based on, tRNA adaptation index (tAl), for example as disclosed in dos Reis et al., Nucleic Acids Res. 2004; 32(17): 5036-5044, and/or a number of effective codons (Nc), as disclosed in Wright et al., Gene, Volume 87, Issue 1, 1 March 1990, Pages 23-29. The

The determination of the tAl may comprise estimating the tRNA levels, which may for example be approximated by the gene copy number (GCN) of tRNA genes that share a specific anti-codon. The determination of Nc may comprise the use of the genetic code to calculate codon homozygosities as a measure of evolutionary selection which tends to reduce the number of possible codons used in an optimized transcript.

In a preferred embodiment of the present disclosure, the gene statistics is a combination, such as a linear combination, of the tRNA adaptation index (tAl) and the number of effective codons (Nc).

It is a strong preference that tAl and Nc is determined for each gene sequence, and that they are used for estimating the gene optimization (*genopt*), such as by calculating a linear combination thereof. The *genopt* statistics may thereafter be summarized across species used in the analysis through their homolog group assignments, using for example the mean or median values.

Alternatively or additionally, the gene statistics (*genopt*) may be based on other performance measures, such as transcription profiling, as performed with RNA-seq, or protein quantification as measured by mass spectrometry. If the method is directed at generating environmentally responsive promoters and/or terminators, the gene statistics (genopt) may be calculated from the relative expression calculated between two or more relevant growth conditions by any or a combination of: tAl, Nc, RNA-seq and/or mass spectrometry.

In an embodiment of the present disclosure, the present method comprises a step of identifying, for each homolog group, a set of functional DNA contexts comprising, for each gene sequence of respective homolog group. The functional DNA context may for example be a DNA sequence upstream of the start codon of the gene sequence and/or downstream of the stop codon of the gene sequence. Typically, for the generation of synthetic promoters, the functional DNA context is preferably a DNA sequence upstream of the start codon (e.g. ATG) of the gene sequence, while for the generation of synthetic terminators, the functional DNA context is preferably a DNA sequence downstream of the stop codon of the gene sequence. Alternatively or additionally to identifying the gene translation start sites (ATG), the transcription start site may be identified, however this is typically not required.

In an embodiment of the present disclosure, the functional DNA context is, for each gene sequence, an upstream DNA sequence, said upstream DNA sequence being upstream of the start codon of the gene sequence, in the respective genome. Preferably wherein said DNA sequence has a length greater than the expected length of the promoter in said given genome.

In another embodiment of the present disclosure, the functional DNA context is, for each gene sequence, a downstream DNA sequence, said downstream DNA sequence being downstream of the stop codon of the gene sequence, in the respective genome. Preferably wherein said DNA sequence has a length greater than the expected length of the promoter in said given genome.

In an embodiment of the present disclosure the length of the upstream and/or downstream DNA sequences may be, or be based on, the average intergenic distance between divergently transcribed genes and/or represent a sequence length equal to or larger than the expected length of the functional promoter/terminator. Additionally or alternatively, other functional DNA contexts can also be used. For example, when modeling gene terminators the DNA sequences downstream (3') of the stop codon may be used to define the functional DNA context.

The length of the DNA sequences used in the functional DNA context typically depends on the species. For example, around 1800 bps may preferably be used for filamentous fungi (e.g. *Aspergillus*) and plant promoters while around 900 bps may preferably be used for yeast species promoters. Consequently, it is a preference that the method of the present disclosure is arranged to use a length of the DNA sequences used in the functional DNA context within the range of 100-5000 bps, more preferably within the range of 300-2000 bps, such as 500 bps or 900 bps or even 1800 bps.

The extracted DNA sequences that define the functional DNA context for promoters/terminators are typically saved in a multi fasta file (upstream.fna) for each gene sequence/annotated genome.

In an embodiment of the present disclosure, the method comprises a step of building, for each homolog group, a multiple sequence alignment (MSA). Each of said MSA preferably consist of the set of functional DNA contexts of each respective homolog group. For each HG, the functional DNA context of each gene sequence will be retrieved. The functional DNA sequences for either a single ortholog or the ortholog and paralogs may preferably be used, and may be saved from each genome in a multi fasta file.

A multiple sequence alignment (MSA) is preferably built for each HG. This may for example be carried out with any one, or a combination, of a number of MSA tools, such as ClustalW or MUSCLE.

In an embodiment of the present disclosure, the method comprises a step of fitting, for each MSA, a hidden markov model (HMM). Preferably said fitting is performed in an iterative process. Typically, wherein the fitting is repeated until convergence and/or until a predetermined number of iterations have been carried out. Preferably, said fitting results in obtaining, for each MSA, a starting consensus sequence.

For the fitting of each HMM, the 5' and 3' ends of the model are typically allowed to vary, typically reduced, through the use of a trimming feature (hmmalign --trim). For each iteration, the current HMM model is typically used to create a new MSA that may then be used to fit a new HMM model. In each iteration, the original set of sequences may be used in the new alignment (i.e. the sequences are not trimmed) but the model may typically be allowed to vary in the number of match states.

Convergence may for example be tested by extracting the consensus sequence from each model (hmmemit) and comparing it to consensus sequences from a number of previous iterations, such as at least 1 or 2 previous iterations, or preferably up to 5 previous iterations. If the same, or at least a significantly similar, consensus sequence is observed, the model optimization process is deemed to have converged. The fitting may be carried out until convergence or up to a maximum number of iterations, for example up to 100 iterations, more preferably up to 300 iterations, even more preferably up to 500 iterations, yet even more preferably up to 1000 iterations, most preferably up to 3000 iterations. Typically, the most probable sequence of each HMM is considered the respective consensus sequence.

In an embodiment of the present disclosure, the method comprises a step of identifying multiple model features of the HMM. Preferably said model features are indicative of regions of high conservation in the starting consensus sequences. The model features are preferably used for the generation of a max-score matrix (FMS) comprising the maximum scores for each model feature for each HMM,

Regions of each model may contain one or more stretches of high conservation. High conservation is typically indicated by high information content (IC) in the match states of the model. As a practical approach to find high-IC stretches, a running mean may be applied for a specific length of match states, such as between 6-9 states. High mean IC windows are typically selected using a threshold, such as 1.5 bits, and the starting positions are recorded. Each window may then be extended, where possible, by merging with other adjacent high-IC windows, typically also allowing for a gap of 1-3 positions. These high-IC and/or merged high-IC windows may be summarized by their consensus sequence (a k-long DNA word or "k-mer") and registered as a model feature. The model features may, due to the merging procedure, have differing lengths.

Each of said model features typically also have a set of scores for each k-long window in the model, and a maximum score from each HMM model. Scores are calculated for each feature in each HMM model. Typically, the maximum score for each feature in each model is identified, and used to define a matrix of maximum scores (k-mers by HMM models), or a feature max-score (FMS) matrix, such as wherein HMM models represent the HGs. The maximum score of a feature is defined from the log-likelihood scores of a k-mer feature in the HMM. These scores are calculated for each possible un-gapped alignment of a k-mer to the HMM. A score is defined by the sum of log-likelihoods for a particular subsequence (k-mer) of consecutive HMM match states. The maximum score, representing the best possible match of the k-mer, is stored in the FMS matrix.

In a further embodiment of the present disclosure, the method comprises a step of forming a statistical model, such as PLSR. Preferably wherein said statistical model may be used for modeling gene statistics as a function of FMS.

The feature max-score matrix or matrix of feature scores (k-mers by HGs), which may contain for example 300 features over 5800 HGs, can be used to fit a model to the gene statistics (genopt). This feature-based model can then be used to predict functionality, such as strength, of DNA designs, such as synthetic promoters and/or terminators. It is a preference the statistical model comprises or consists of Partial Least Squares regression (PLSR), that may be used to model gene statistics *(genopt)* as a function of feature scores (FMS matrix).

In a preferred embodiment of the present disclosure, the parameters (feature coefficients) of a fitted statistical model, such as a PLSR model, are used to identify which k-mer features relate positively (or negatively) to a model's or DNA design's functionality. In the case of promoter design, the gene statistics *(genopt)* relates to gene expression level or promoter activity and k-mers with large positive coefficients in the PLSR model indicate determinants of higher activity.

In a preferred embodiment of the present disclosure, the method comprises a step of identifying activity regulating features. Preferably by calculating a correlation coefficient, for example the Pearson correlation coefficient, between parameters of the statistical model and features scores of the FMS, and recognizing said activity regulating features as model features of which said correlation coefficient, for example the Pearson correlation coefficient, is above a predetermined threshold, such as 0.5.

To identify the features that are determinants of activity, the correlation may be calculated between the coefficients of the statistical model, e.g. the PLSR coefficients, and the feature scores in the FMS matrix. Features that have a Pearson correlation coefficient above a predetermined threshold, e.g. r > 0.5, are preferably considered to be an activity regulating feature, and may as such be considered in a subsequent step for optimization of the starting consensus sequence.

In a preferred embodiment of the present disclosure, the method comprises a step of optimizing, for each HMM, the starting consensus sequence, by, for each activity regulating feature, calculating the gene statistics upon inserting/replacing said activity regulating feature into the starting consensus sequence or a partially optimized consensus sequence. For instances wherein said insertion/replacement leads to an improvement in the gene statistics, such as an increase by a predetermined threshold, the insertion/replacement is preferably accepted, and may thereafter be used for a subsequent round of optimization. For instances wherein said insertion/replacement does not lead to an increase, such as by a predetermined threshold, said insertion/replacement is preferably not accepted, and the current sequence is preferably used for a subsequent round of optimization.

It is a preference that the step of optimization comprises sorting the activity regulating features from highest to lowest correlation to the statistical model coefficient (e.g. PLSR coefficients). For each activity regulating feature, the position in the design where the maximum feature score was observed, and preferably any other positions that score above a predetermined feature score threshold (such as a predetermined fraction of the maximum feature score), are tested for insertion/substitution with the feature k-mer.

Using the statistical model, e.g. the PLSR model, if the overall design improves its predicted gene statistics *genopt,* by more than a defined margin, e.g. (Sₒₚₜ - Sₒᵣᵢ) > 0.1, then the feature insertion/substitution is preferably accepted. Typically, subsequent activity regulating features are less likely to result in accepted substitutions when starting with features with the highest correlation to the statistical model coefficient, e.g. PLSR coefficients.

In an embodiment of the present disclosure, the performance parameters are selected from the group consisting of: transcription rate, preferably based on tRNA adaptation index and/or number of effective codons; transcription profiling, preferably based on RNA sequencing data; and protein quantification, preferably measured by mass spectrometry.

In one embodiment of the present disclosure, the species are selected from any one of *Aspergillus, Saccharomyces, Kluyveromyces, Komagataella, Barnettozyma, Cyberlindnera, Phaffomyces, Physcomitrium, Starmera, Wickerhamomyces, Yarrowia* and/or *Trichoderma.*

In one embodiment of the present disclosure, the species are fungi species, such as belonging to *Aspergillus, Kluyveromyces, Saccharomyces, Yarrowia* and/or *Trichoderma.* For example, the species is *Aspergillus niger, Kluyveromyces lactis Saccharomyces cerevisiae, Yarrowia lipolytica, Trichoderma nicaragua* and/or *Trichoderma reesei.*

In one embodiment of the present disclosure, gene sequences of genomes of at least five species are obtained.

In one embodiment of the present disclosure, the number of homolog groups is at least 80% of the number of coding genes or of the number of predicted coding genes in any one of the species, such as at least 90%, such as at least 95%.

In one embodiment of the present disclosure, the gene statistics is calculated for each gene sequence of the homolog groups. In some instances, the gene statistics is calculated based on tRNA adaptation index (tAl) and/or the number of effective codons (Nc). In some instances, the gene statistics is based on transcription profiling, or protein quantification or relative expression calculated between 2 or more relevant growth conditions. For example, the expression of a given gene sequence or gene in a species, for example a microorganism, can be high when the cells of the species are growing in a nutrient-rich environment, and low when the cells of the species are growing in a nutrient-deprived environment. Some gene sequences or genes also display a higher expression level under conditions of high osmosis or under hypoxia.

In one embodiment of the present disclosure, the gene statistics is, for each gene sequence, a linear combination of the tRNA adaptation index and the number of effective codons.

In one embodiment of the present disclosure, the expected length of the promoter and/or expected length of the terminator in a given genome corresponds to the average intergenic distance between divergently transcribed genes in said given genome. The skilled person knows how to find the average intergenic distance between divergently transcribed genes in a given genome.

In one embodiment of the present disclosure, the expected length of the promoter and/or expected length of the terminator is at least 800 bps for eukaryotes, such as at least 900 bps, such as at least 1000 bps. In some embodiments, the expected length of the promoter and/or expected length of the terminator in a given genome is between 500 and 1000 bps, between 600 and 900 bps, or between 700 and 800 bps.

In one embodiment of the present disclosure, for each iteration of the HMM, the HMM is used to create an updated MSA that is used to fit an updated HMM.

In one embodiment of the present disclosure, the fitting is repeated until convergence that is defined as no change of the consensus sequence, such as the most likely outcome of the HMM, for a predetermined number of iterations, such as five.

In one embodiment of the present disclosure, the regions of high conservation are defined as regions of high information content in the match states of the model.

In one embodiment of the present disclosure, the regions of high information content in the match states of the model are identified by:
a) performing a running mean for a predetermined length in match states of the model, such as between 6 and 9 match states, and
b) identifying regions with a value above a predetermined threshold, such as 1.5 bits.

In one embodiment of the present disclosure, merged regions of high information content in the match states of the model are identified by merging regions of high information content in the match states of the model that are separated by at maximum a predetermined number of positions, such as between 1 and 3.

In one embodiment of the present disclosure, the model features are identified by summarizing the consensus sequence of the regions of high information content and/or the merged regions of high information content in the match states of the model.

In one embodiment of the present disclosure, the FMS is generated by identifying the maximum score for each model feature in each HMM model.

In one embodiment of the present disclosure, the statistical model is configured to predict performance statistics, such as functionality and/or strength, of promoters or terminators.

In one embodiment of the present disclosure, optimization of the starting consensus sequence is carried out from the activity regulating feature with the highest correlation to the parameters of the statistical model to the activity regulating feature with the lowest correlation to the parameters of the statistical model.

In one embodiment of the present disclosure, the activity regulating feature is tested for insertion/replacement at the position of the FMS and 85% of the FMS of that activity regulating feature.

In one embodiment of the present disclosure, the statistical model is a partial least square regression model.

In a preferred embodiment of the present disclosure, the method comprises the steps of:
i. obtaining gene sequences, such as functional sequences and/or coding sequences, of genomes of multiple closely related species,
ii. identifying at least one homolog group, each homolog group comprising multiple gene sequences that are homologs,
iii. identifying gene statistics for each gene sequence of the homolog groups, wherein the gene statistics are based on performance parameters and are a prediction of the strength of promoters or terminators of each respective gene sequence,
iv. identifying, for each homolog group, a set of functional DNA contexts comprising, for each gene sequence of respective homolog group:
   ∘ in the case of generation of a synthetic promoter: an upstream DNA sequence, said upstream DNA sequence being upstream of the start codon of the gene sequence, in the respective genome, and having a length greater than the expected length of the promoter in said given genome, or
   ∘ in the case of generation of a synthetic terminator: a downstream DNA sequence, said downstream DNA sequence being downstream of the stop codon of the gene sequence, in the respective genome, and having a length greater than the expected length of the terminator in said given genome,
v. building, for each homolog group, a multiple sequence alignment (MSA), each MSA consisting of the set of functional DNA contexts of said homolog group,
vi. iteratively fitting, for each MSA, a hidden markov model (HMM), wherein the fitting is repeated until convergence and/or until a predetermined number of iterations have been carried out, thereby obtaining a starting consensus sequence for each MSA,
vii. identifying multiple model features of the HMM, the model features being indicative of regions of high conservation in said starting consensus sequences, and generating a max-score matrix (FMS) comprising the maximum scores for each model feature for each HMM,
viii. forming a statistical model, such as PLSR, for modeling gene statistics as a function of FMS,
ix. identifying activity regulating features, by calculating a correlation coefficient between parameters of the statistical model and features scores of the FMS, and recognizing said activity regulating features as model features of which said correlation coefficient is above a predetermined threshold, such as 0.5, and
x. optimizing, for each HMM, the starting consensus sequence, by, for each activity regulating feature, calculating the gene statistics upon inserting/replacing the activity regulating feature into the starting consensus sequence or a partially optimized consensus sequence,
   ∘ where said insertion/replacement leads to an improvement in the gene statistics, such as an increase by a predetermined threshold, accepting said insertion/replacement,
   ∘ otherwise said insertion/replacement is discarded.

In an embodiment of the present disclosure the method further comprises any, or a combination, such as all, of the steps of:
- synthesizing an artificial sequence comprising: the synthetic promoter and a target gene sequence downstream of the synthetic promoter; or the synthetic terminator and a target gene sequence upstream of the synthetic terminator;
- introducing said artificial sequence in a target genome;
- measuring transcription levels from the target gene sequence; and/or
- comparing the transcription levels measured previously measured with the transcription levels obtained from the same target cording sequence with the native promoter or with the native terminator, thereby determining the strength of the synthetic promoter and/or terminator relative to the strength of the native promoter or of the native terminator,
wherein the said steps of synthesizing, introducing, measuring and comparing are preferably preferred in said order, and/or preferably wherein said steps of synthesizing, introducing, measuring and comparing are performed after the step of optimizing.

Thus, in an embodiment of the present disclosure, the method further comprises the steps of:
xi. synthesizing an artificial sequence comprising: the synthetic promoter and a target gene sequence downstream of the synthetic promoter; or the synthetic terminator and a target gene sequence upstream of the synthetic terminator;
xii. introducing said artificial sequence in a target genome;
xiii. measuring transcription levels from the target gene sequence; and/or
xiv. comparing the transcription levels measured in step iii) with the transcription levels obtained from the same target coding sequence with the native promoter or with the native terminator, thereby determining the strength of the synthetic promoter and/or terminator relative to the strength of the native promoter or of the native terminator.

In one embodiment of the present disclosure, the strength of the synthetic promoter and/or of the synthetic terminator is at least 100% of the strength of the native promoter and/or of the native terminator, respectively, more preferably at least 110%, such as at least 120%, such as at least 130%, such as at least 140%, yet more preferably at least 150%, such as at least 160%, such as at least 170%, such as at least 180%, such as at least 190%, even yet more preferably at least 200%, such as at least 250%, once even yet more preferably at least 300%, such as at least 350%, most preferably at least 400%, such as at least 450%, such as at least 500%.

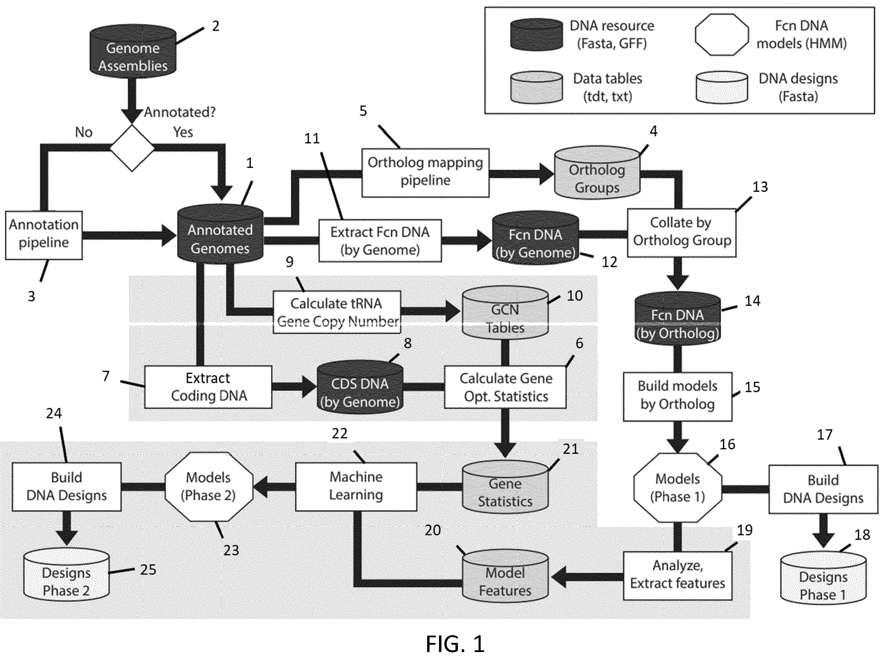

In the following, reference will be made to the drawings, which are exemplary and are intended to illustrate some of the features of the presently disclosed methods for generation of synthetic promoters and sequences obtained by such methods, and are not to be construed as limiting to the presently disclosed invention. As shown in Fig. 1, the method for generating a synthetic promoter and/or terminator typically comprises obtaining gene sequences (1), such as functional sequences and/or coding sequences, of genomes of multiple closely related species. The gene sequences may be received from publicly available databases, typically as genome assemblies (2). The gene sequences may be available with annotation, however this is not a requirement. Instead, for instances wherein the gene sequences are not annotated, it is a preference that the gene sequences are annotated through an annotation pipeline (3).

Based on the gene sequences, at least one homolog group (4) is identified, e.g. through a homolog/ortholog pipeline (5), wherein each homolog group comprises multiple gene sequences that are homologs.

Further, the method comprises calculating (6) gene statistics (21) for each gene sequence of the homolog groups, wherein the gene statistics are based on performance parameters and are a prediction of the strength of promoters or terminators of each respective gene sequence.

Codon optimization may be used as a measure of promoter activity and mRNA levels in addition to its ability to for example predict protein translation rates and subsequent protein levels. This includes extracting coding DNA sequence (7) to obtain the CDS DNA by genome (8).

Alternatively or additionally, the gene statistics may be calculated from the tRNA adaptation index (tAl). The determination of the tAl may comprise estimating the tRNA levels, which may for example be approximated by the gene copy number (9, GCN) of tRNA genes that share a specific anti-codon, resulting in GCN tables (10).

Alternatively or additionally, the calculation of the gene statistics may include the determination of Nc, comprising the use of the genetic code to calculate codon homozygosities as a measure of evolutionary selection which tends to reduce the number of possible codons used in an optimized transcript.

In a preferred embodiment of the present disclosure, the gene statistics is a combination, such as a linear combination, of the tRNA adaptation index (tAl) and the number of effective codons (Nc).

The presently disclosed method further comprises extracting/identifying (11), for each homolog group, a set of functional DNA contexts (12) comprising, for each gene sequence of respective homolog group. As described elsewhere herein, the functional DNA context may be an upstream or a downstream sequence, depending on whether a promoter or a terminator is generated.

The presently disclosed method further comprises building (13), for each homolog group, a multiple sequence alignment (14, MSA), each MSA consisting of the set of functional DNA contexts of said homolog group,

The presently disclosed method further comprises iteratively fitting (15), for each MSA, a hidden markov model (HMM) (16), wherein the fitting is repeated until convergence and/or until a predetermined number of iterations have been carried out, thereby obtaining a starting consensus sequence for each MSA. The HMM may be used to generate synthetic promoters and/or terminators sequences (17), according to steps i. - vi. of the presently disclosed method. This may be referred to as synthetic promoters and/or terminators having a phase 1 design (18), phase 2 designs may be generated by also carrying out steps vii to x of the presently disclosed method. SEQ ID NO: 1 to 8 have all been generated used the phase 1 of the presently disclosed method.

The presently disclosed method further comprises identifying multiple model features of the HMM (20), the model features being indicative of regions of high conservation in said starting consensus sequences, and generating a max-score matrix (FMS) comprising the maximum scores for each model feature for each HMM.

The presently disclosed method further comprises forming a statistical model (22), such as PLSR, for modeling gene statistics as a function of FMS.

The presently disclosed method preferably further comprises identifying activity regulating features, for example as part of the statistical model (22), by calculating a correlation coefficient between parameters of the statistical model and features scores of the FMS, and recognizing said activity regulating features as model features of which said correlation coefficient is above a predetermined threshold, such as 0.5.

The presently disclosed method further comprises optimizing, for each HMM, the starting consensus sequence, by, for each activity regulating feature, calculating the gene statistics upon inserting/replacing the activity regulating feature into the starting consensus sequence or a partially optimized consensus sequence, and
∘ where said insertion/replacement leads to an improvement in the gene statistics, such as an increase by a predetermined threshold, accepting said insertion/replacement,
∘ otherwise said insertion/replacement is discarded.

The resulting functional DNA models (23) may thereafter be used in order to build optimized DNA designs (24). This may be referred to as synthetic promoters and/or terminators having a phase 2 design (25).

The optimized DNA design may, as discussed elsewhere herein, either be synthetic terminators or promoters. By the use of said optimized DNA designs a desired activity level in for example protein production may be achieved, typically it may be desired that the production is maximized.

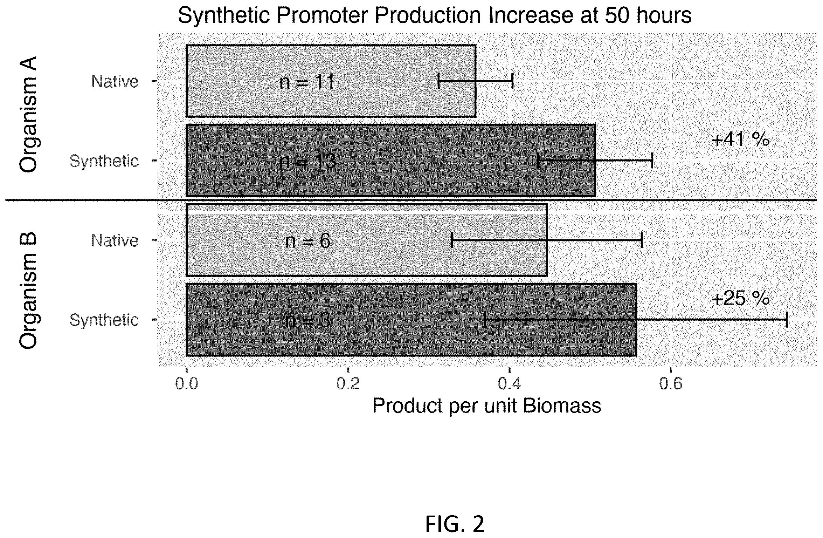

Fig. 2 shows results of comparison between phase 1 synthetic promoters and/or terminators (18 in Fig. 1), e.g. generated by carrying out steps i-vi of the presently disclosed method) with corresponding native promoters and/or terminators. Two separate organisms having the native promoter and a synthetic promoter, generated by an embodiment of the presently disclosed method. As described elsewhere herein, HMM have been generated, and the consensus sequence have been obtained corresponding to the phase 1 design. It can be seen that the synthetic promoter generated according to phase 1 of the presently disclosed method (steps i.-vi. of the presently disclosed method) led to a production increase of 41% for Organism A, and 25% for Organism B (in terms of product per unit biomass). SEQ ID NO: 1 to 8 discloses multiple sequences generated by steps i.-vi. of the presently disclosed method.

A further aspect of the present disclosure relates to a statistical model for generation of synthetic promotes and/or terminators, the model generated according to any of the methods disclosed herein.

Yet a further aspect of the present disclosure relates to a data processing system for generating strong synthetic promoters and/or terminators, the system comprising an input device, a central processing unit, a memory, and an output device, wherein said data processing system has stored therein data representing sequences of instructions which when executed cause a method disclosed herein to be performed for the generation of synthetic promoters and/or terminators, the memory preferably further comprising at least a statistical model.

In one embodiment of the present disclosure, the statistical model is stored in a server, and the input and output devices are a client, the client and server being connected via data communication connection.

In one embodiment of the present disclosure, the client is selected from a personal computer, a stationary PC, a portable PC, a hand-held computing device such as a smart phone.

In a further aspect, the present disclosure relates to a computer software product containing sequences of instructions which when executed cause the method disclosed herein to be performed, such as for the generation of synthetic promoters and/or terminators.

In yet a further aspect, the present disclosure relates to an integrated circuit product containing sequences of instructions which when executed cause the method disclosed herein to be performed, such as for the generation of synthetic promoters and/or terminators.

In yet a further aspect, the present disclosure relates to a data processing system for identifying synthetic promoter and/or terminator sequences, the system comprising an input device, a central processing unit, a memory, and an output device, wherein said data processing system has stored therein data representing sequences of instructions which when executed cause the method disclosed herein to be performed, such as for the generation of synthetic promoters and/or terminators.

In yet a further aspect, the present disclosure relates to a method for expressing a protein of interest in a cell, the method comprising:
- providing a cell comprising a gene sequence encoding the protein of interest,
- inserting a synthetic promoter, and/or a synthetic terminator, obtained by the method disclosed herein,
- incubating the cell in a medium,
whereby the cell expresses the protein of interest, preferably wherein said steps are performed in the order of providing, inserting and incubating.

Thus in an embodiment of the present disclosure the method comprises:
I. providing a cell comprising a gene sequence encoding the protein of interest,
II. inserting a synthetic promoter, and/or a synthetic terminator, obtained by the method disclosed herein, and
III. incubating the cell in a medium,
whereby the cell expresses the protein of interest.

In a preferred embodiment of the present disclosure, the synthetic promoter is inserted upstream of said (target) gene sequence, and/or the synthetic terminator is inserted downstream of said (target) gene sequence. Preferably in such a way as to control the expression of the (target) gene sequence, for example in order to increase the production/yield of the protein of interest.

### Examples

The invention will in the following be described in greater detail with reference to the accompanying drawings. The drawings are exemplary and are intended to illustrate some of the features of the presently disclosed methods for generation of synthetic promoters and sequences obtained by such methods, and are not to be construed as limiting to the presently disclosed invention.

Example 1: Preparation of spore solutions and inoculation of plates:
1. Grow fungus on plate until sporulation
2. Harvest the plate with 5mL MQ 0.9% NaCl solution
3. Filter the spores through miracloth
4. Centrifuge: 8000g for 3 min. Decant supernatant, and resuspend in 0.5-1mL of MQ-water
5. Create a plate-layout table containing; well number, fungal strain and specie specifics

Make dilutions 1:10, 1:100, 1:1000 in 1 ml tube and count the spores in the counting chamber at appropriate concentration (20-40 spores per large square). Calculate spore concentration in the stock solution, and how much is needed to obtain 1 mL of 1x10⁸ spores/ml.

Make a 1x10⁸ spores/ml spore solution. Vortex.

Add 100µL of spore solution into 9.9 mL of appropriate media. Vortex.

Add 1.5mL to each well of a deep 48-well microtiter plate (a 48-MTP "Flower plate" from m2p)

Seal plate with a breathable seal.

Start a BioLector II run:
- 1.5 mL final volume and 1000 rpm
- Set humidistat to 85%
- Set Biomass filter gain a value of 3
- Set Fluorescent protein filter gain to 7

Run for 3 days, 85% humidity (37C for *A. nidulans,* 30C for other species)

### Example 2: Generation of synthetic promoters and/or terminators

Synthetic promoters and/or terminators were generated by carrying out the following steps:
i. obtaining gene sequences, such as functional sequences and/or coding sequences, of genomes of multiple closely related species;
ii. identifying at least one homolog group, each homolog group comprising multiple gene sequences that are homologs;
iii. identifying gene statistics for each gene sequence of the homolog groups, wherein the gene statistics are based on performance parameters and are a prediction of the strength of promoters or terminators of each respective gene sequence;
iv. identifying, for each homolog group, a set of functional DNA contexts comprising, for each gene sequence of respective homolog group:
   ∘ in the case of generation of a synthetic promoter: an upstream DNA sequence, said upstream DNA sequence being upstream of the start codon of the gene sequence, in the respective genome, and having a length greater than the expected length of the promoter in said given genome, or
   ∘ in the case of generation of a synthetic terminator: a downstream DNA sequence, said downstream DNA sequence being downstream of the stop codon of the gene sequence, in the respective genome, and having a length greater than the expected length of the terminator in said given genome,
v. building, for each homolog group, a multiple sequence alignment (MSA), each MSA consisting of the set of functional DNA contexts of said homolog group,
vi. iteratively fitting, for each MSA, a hidden markov model (HMM), wherein the fitting is repeated until convergence and/or until a predetermined number of iterations have been carried out, thereby obtaining a consensus sequence for each MSA.

The generated synthetic promoter and/or terminator was compared to that of a native promoter and/or terminator in order to confirm that the desired expression is achieved

Results: Generated synthetic promoters and/or terminator are given in SEQ ID NO: 1 to 8. Fig. 2 shows the comparison in protein production between a generated synthetic promoter and the corresponding native promoter.

Conclusion: The synthetic promoters and/or terminators had a desired level of protein production, compared to the native promoter and/or terminator.

### Example 3: A screen of selected promoters in Komagataelle phaffii

Aim: To investigate whether the activity in the synthetic phase 1 designed promoters is higher than in native promoters.

Materials and methods: A screen of selected promoters was performed in *Komagataelle phaffii* to assess a representative number of promoter designs compared to their native sequences from *K. phaffii.* Ortholog groups were identified from four *Komagataella* species (*K. phaffii, K. pastoris, K. pseudopastoris, K. populi*). In total 5868 ortholog groups (OGs) were identified and assessed for the strength of their expression by the codon statistics method described in the application. From this set of 5868, 270 OG's were selected as the strongest expression candidates for this genus, and phase 1 models (HMMs) were built for all 270 selected orthologous promoters. From the 270 phase 1 models, 61 were selected for screening in *K. phaffii.*

For each of the 61 candidates, a phase 1 design and a native *K. phaffii* promoter DNA sequence was synthesized (Twist Biosciences). The library of 122 promoters was then genetically engineered into *K. phaffii* GS115 cells by integration using plasmid-based CRISPR/Cas9 method, including Nat resistance gene, and used to express yeGFP integrated downstream from these promoters. Integrations were multiplexed in 6 separate transformations using electroporation where roughly 20 sequences were combined from the library. All resulting transformants were plated on petri plates containing YPD agar + 200µg/mL nourseothricin and incubated for 3 days at 30 °C. 285 clonal colonies were picked at random using a QPix colony picker (Molecular Devices LLC, U.S.A) and transferred to 96-well greiner plates with liquid YPD media. These plates were then incubated overnight at 30 °C with agitation (700 rpm). The library of transformants was then sequenced using primers for the integration site in order to identify which promoter sequence had been integrated in the transformation. Based on the sequencing results, 261 transformants were identified, representing 96 distinct promoters (76%, 50 native and 43 phase 1 designs) from the original library of 122 promoters (61 pairs). 64 of the 96 promoters were recovered from independent transformants at least twice.

The library of transformants was then replicate plated onto Q-Tray plates containing YPD agar with a plate replication robot (ROTOR HDA Singer Instruments). After incubation for 1 to 2 days at 30 °C, fluorescence analysis of the arrayed colonies was performed using a SpectraMax iD3 multi-mode plate reader with excitation at 435 nm and measured emission at 480 nm (Molecular Devices LLC, U.S.A).

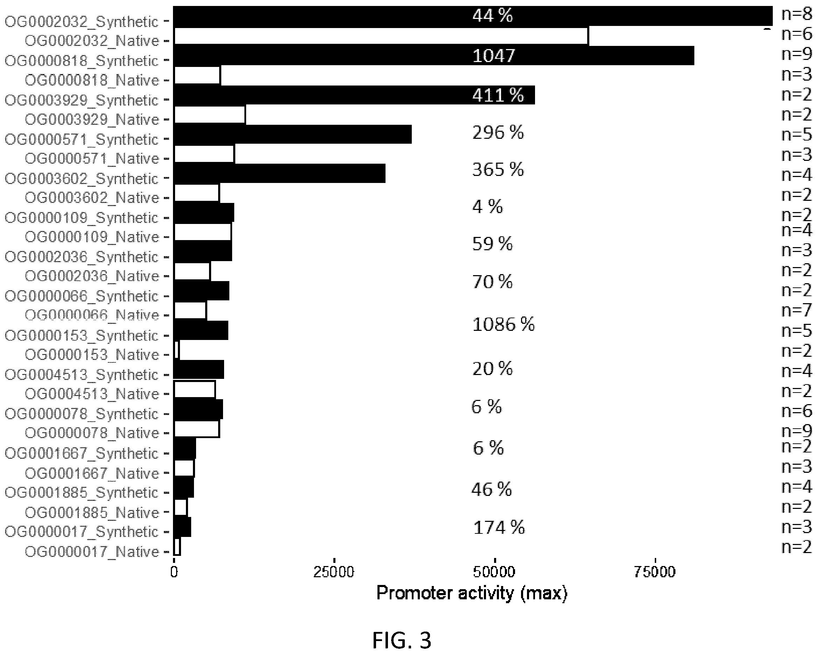

Results: In total, 35 promoters were observed in both the native and synthetic phase 1 version and could be compared directly for their fluorescence. In 26 of the 35 cases the maximum observed fluorescence was greater in the synthetic version than in the native version (74%). Increased fluorescence was observed in 14 of the 18 cases where both the native and synthetic versions were observed 2 or more times in the transformant library. Of the 9 pairs where the synthetic design performed lower than the native, the percent decrease ranged from 1-84% decrease while the 26 improved designs ranged from 4-1086% increase in maximum performance (Figure 3).

Conclusion: The inventors of the present disclosure have realized that the activity in the synthetic phase 1 designed promotors is in 14 of 18 cases higher than in the native promotors.

### Example 4:

Aim: To investigate the improvement between synthetic phase 2 and phase 1 designs.

Materials and methods: Features were extracted from the phase 1 models of example 3 and were used to make improved synthetic designs for 241 orthogroups from the selected orthologous promoters representing our predictions of the highest expressed genes in the *Komagataella* genus. The PLS model scores for these phase 2 designs were predicted.

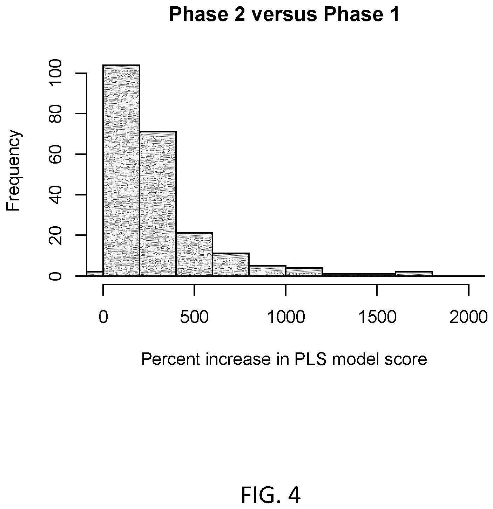

Results: The estimated improvements of synthetic phase 2 designed promoters when compared to synthetic phase 1 designed promotors, showed a projected improvement of 200% based on the median percent change (Figure 4).

Conclusions: The inventors of the present disclosure have realized that the activity in the promoters of the synthetic phase 2 design have a projected improvement of 200% based on the median percent change.

### Sequence overview

SEQ ID NO: 1 Synthetic constitutive promoter BBa_K3046001|PLEAPglaA_2|low: 
CGTCCAGCTCCATAAGCTGGGTATCCCACTCCGATTGACTATGATTCATGCTAGCT
GGAAGTCAATCTCGGGTAGTGTTATACTGGACGTATCTCGGCATGACAGGCGATA
AGTGGAAGGATGCATGCACGGAATGCAAAGCATAGGATTGATCTACGGAAATGGG
ACTCCCGATTTGTCGCCAAACGATGCACCGTGGCCCACGCATGACCCCTACTTTT
GCGAGTTGCTTGATACCTAGTCGGACCCAAAAAACATCCGAGTTCACAGCCTGTG
CTTAGTCCGAAGGCCTCAGATGGCGAATGTACGTGGAGAATATGGTTGAATCTTA
AGAACGTGAGGTTAATGGTTGGACCACGTGGGCTCACCTCACGCGCAGCTGCGG
AGCCGCTAGAATCGTCGTGTTGATTGTAAACTTACATCCCGCCATTAGACTAAAGT
CGGCATTGGTAATGTCAGGGAAAACCGTGTGTCGGATAATGCCCGCGATCCTATG
CGCTTTCTACTCGGTATCGCGTGAGCTTGCACTGAACGATCAGCACACAAACCCT
GTGCACTCGCTATACACTAACAACAAAGTTTTAAAAGAGCTCGCTTTCGCAATGCG
CAGAGACACTGAGTGCAGCTGCCTCCTTCTTCAGTCTCCACCTCTCCTTTATATTA
TATTTGCGCAGTCGCCTCGC
SEQ ID NO: 2 Synthetic constitutive promoter BBa_K3046002|PLEAPsonB_1|low: 
TTTTTTGTTGTCCAATGTAGTTTGGGCTGTTCCTTCACATGACAGCAGTGTTGGGC
TAGAGAGAGAAGAGGATCTGATTGCGAGAATCCAGGATGCGTCGACGGCGACAG
GCGACAGTCCCTCATCCGTACTGTTACTCTGATACGTGCCTCGGAATCCTAAGGG
GCTTGAATCAACAGAATCAGCAGACAAGATGATCGCAGCTTCTAGGATGCAGTCA
GCTGACCGGATCTGGCTCCGTAATTCAGGCTTCGGAGGGGCATCAACGGCACGT
GACTTAATGCGGCGGTACCACACCCGGTGGTCCCTCGGAAAACCCTCCAGTACC
AGCGGTACCGTTCCAGATCTGACCTTACCGGCTGTTCCGCAGCCGTAAGCGAGG
CAGGCAGCGGTAACACTACCCGTCCTTCGCTCTCTCTCTCCCTTCTCTTCTCTTCA
TCTCTCCGCTTTTTCCCTTCACCGTCGCGCGTCCTCTTCTCTTTCCCCTCACCTCT
TCATCTTTTTCTCTCTCGCTTTCGACCCTTCCTTTCGGACTTGTTCACATCCTAGCT
TTGACGGTGACTTCCCGTCTGTAGCTGGTGTGGCCCTTTTTTTTTGCTTTTCTAGA
TCCCTTCGCAAAACAAAACACCTTTTCTCTGTTTTTCTGCGCGTCGAGATCTCTCTT
CGCGAGCCACTCTTTCGCCTCTACTTTGTGCTAGCGCACTGCGATCGATCTTTTC
GCCTACTGTTATTTCTTGATACATCAGCTTTAGTTATCGTCGTTTTACGAGCTTTCC
AACTTTTCGGTCGCCGCCCTCCTTCGAACGGATGAATCCATAATCCGCAGCAACA
ACAAACAGCAA
SEQ ID NO: 3 Synthetic constitutive promoter BBa_K3046003|PLEAPgpdA_1|high: 
CACCATGGTAAGCGCGTGATCGCTCGTGTGCCTAACCGGTGACTCTTTCTGGAGT
GCGGAGAGAACGAGGGACGCCTAGAGAGAAGCCTAAGCGATCCAGCCAGTGCC
GGCAAAGAGGAGGGGCAGTGGATCATCGTTATTCCGTGCCGGGCTCACGGGCC
GAAAGCGGAAAGGCCGGAGTGCCCCTCATTTTTGGTGGGAAGCGTGGGATGGTG
GTGTTCCATTACATCATCCCTTCCTTTGGGGCAATTCCCCTCCTCCGCGGCAGTAT
GCGGGTAGGAACGTAAAAACCGGGGGATAGACCCGTCGTCCCGAGATCGCCTTC
CCAATAACCTAGGTAGAACAGCCCAATTGATTCCGAAAAGCTGGCAAAAGATTCA
CGAGATGGTAGGAGTGAGTACCCGGCGTGATGGATATGGAGCACGCTCATTGGC
CCATCCGGCAGCTGCCGAGGGGAGGCAGGAGTCCCAAATATCGTGAGTCTCCTG
CTTTGCCCGGTGTATGAAACCGGAAAGGGCTGCTGGGGAGCTGGGGAGCGGCG
CAAGCCGGGAACACAAGCTGACAATTGACCCATTTCCTGCACATTGGCAGAGCTT
GCGGTAGCTTTTGCCCCGTCTGTCCGCCCGGTGTGCGGACTGGCGCGAGGAAGA
GGTGACCTGGTCGTTGCGTCAATCCATTCTGTCGCTATCCTTTTTCTACTTCCCCT
CCCCCGCCGCTGAACTTTTCTTCTCTCTTCTTCTCTCTTTCTCTCTTCATCATCATC
TCCCTTCTATCTCCATCTTCTTTTCATCCAATTACCTTAAAGTAAGTCCTCTCCCCC
CCATCCTTCCTCTTCATCTTCTTCATCCCCTTGTACTTTCCTTCCTCTTCCCTCCCT
CACGAAGCTTTACTAACAGCTACCCCGCCCCCAACAGACACATCTAAAC
SEQ ID NO: 4 Synthetic constitutive promoter BBa_K3046004|PLEAPgpdA_2|low: 
CAACAATATAAAATCGTGATCACTCCTATGGACGGCCGGTGACTCTTTCTGGAGC
ACCGAGAGAACATGGGGGGCCAGAGGTCGTCCGCCTGGGGGACTTTCAGCGAC
ACCAACGAAGAGGAGCAGGGGATTATCATTCCTACAGACCGGTGGAACCGGCCG
AAAGCGGCAAGGCCGGGGTGCCCCTCGTTGCCTTATGCAAGTAGGGGCCGTTTG
TCTTCTGTTACATCATCCACTCCCCGACTTGCACCCCCCACTTCCGCAATAGTTAT
CGCCGCGAATTGGGAAACCCGAGGAAAGAACCTGTCTTGCCGACATGGAGATGC
TCATAACCCAGGTACATGAGGCCAAGTGATTCCGACATTCTGGCAAAAGGTTCAC
GAGTTAGTATCAGTGTGTACCCGGCGTGCTAGATGCGAGGCGCGGTTATTGGCC
CAGCCGGTAGCAGCCGAAGACAGACAGGTCTGCCAAATATCGTGAATCTCCTGCT
TTGCCCGGTGTATGAAACCGGAAGAGGCTGCTTAGGAACGGCGGGGCGGCGTAA
GTCGGGAAACTAAGCTGACATTTAACCCTTTTCCCGTACACTGGAAGCGCTTCTTG
TGGCTTTTGCCCCGTCTGTCCGCCCGGTGTGTTAACGGGCGCAAGGTGTTAGTG
ACCAGGTCGTTGCGTCAATCAATTGCCTTGCTATCCTTTTTCTTCTTCCGCTCCCC
CACCGTCGGACTTTTCTTTCCTCTTTTACTCTCTCTTCCTCTTCATCATCCTCTATC
CTCTATCTTCTTCTTTTTATCATCCTTATCACTAAAAGTGAGTCCCCCTCGGTGAAT
TGACCTTTTCGAACTCCTCCATCATAGCCGCTTCCCCTTCTCCCACTCTCCGTCAC
GTAGCTCTGCTAACAGCTACCCCGCCCTAAACAGACACATCCACAC
SEQ ID NO: 5 Synthetic constitutive promoter BBa_K3046005|PLEAPmstA_1|high: 
GTTGATCAGGGTCCAAGAAGCCTATTTCGTGATCGGAAACCCAAAAGGGAGGCCC
TAACCGGATCCGGCTAGTGCTGGGACCCGAGATTGTGGGGAATCCATCCCGGGC
GAACACGTGGTAGGCTGAATTTTCCGACCCCTGTCCATTCCCGCCAATCAGGGCG
GGTCCTACTCGGGCTTGTCCGGCTATTACCAGCCTAGCCAGCCCGACTGTCAGTC
CGGCGCTGACCCGAGCCTCTGTTTGGCCAGATCCCCCTGGCTTGTCCCAACCAC
AGCGAACTAGCCAAGGACATAAGTCCGCTATGCCAAGTGGCTGGGGTTAGCTTGA
GTGTGCCCTCCTAGACTGTCGTCGCCCACTCCGGGGTTGGATACCTTATAGTCAG
CCGGAGGAAGGTAGTACAGTACACCCGCCCCTAGCCATGCCAATGTAACCTATAA
ACTGAAATCCGGCTCCTCAGCCCCATGATTGGCCGGCAATCATTTTTCTCCTGAA
GTAGGCAATCCTAATCAGGACTGGTTGTAATTTTGGCCCCTGTGCGTCCCCTCGG
CCAGCCCCCAGGACGATGGCAAATCGAGGTTCACGGCATTGGCTGGTAAATCGT
CCCATCTAGTCGGGGCAAGACGAGAAAAAAAACAGGTCAAATGAAAATGGAGTCA
TGAACTCATGGAGCCGAGTGTTAGGTGCATTCCCCGTGGGCAATTCCCCGGACC
CGGTTCTGGGCCGATTCACGCCATGGGGAAACCGCGAGTCTATGCAGCTCCGTT
TCTCCCTCAACGGATTATAAAACCCCCTGCTTCCCCCCCCCTCTTTTCTCCCCTGC
TTCTCTTTCCCTCTTCTTCTTCGATCTTCCTCCTTACCTTCACTCTCCTTCCCTTTTT
 
GGTTGCCATAGCGGCTTCTCCCCCCCTCCCCAGCCCTCCCTTTCCTTCTCTCTCT
TATTCTCTTACTTTACAGAGCATCACGCAGTCTCCTCCTCGACATCAGTCAC
SEQ ID NO: 6 Synthetic constitutive promoter
   BBa_K3046006|PLEAPunk_1:An07g08210|low: 
ATTGATGAGGGGACGAATGCCAAGGCTGGAGCATAATTTATTTACATATTGAGATT
GATGACTATATCCTTTCTTAGAACGGACTGAATATCATTGGAGCCTATGCTGAGTT
ACACTTAAATAGTTATCCTGCATTATGGAAAAACAATGTCGATGGTTGGGTAAATA
GTTATGAGCAGGTTAGCAATCATTTCTCTGATGAACTTGTCTGCTTAGATCTGTGA
GTGGATCTTGTTATCCGTTGATCAATCGAACATACTCAGGGAATCAACCACACCGT
ACATACTATTCTACTAATCCTAGTAACTCAGCAATAACGATTATATAAATTATCTCTT
ACTCCGTAATCAGAAGTGCCATCGTGGCACCTGAGTGGATCTGTGGCTGGAATGA
TGATCGACGTCACTAGATAAGGAAGTGTGGCGCAGTCACCTGACCGCGCCCACC
ATACCCGATCGGGAGATTTGTTTTCGGGGAGGCTGGGAAACTCGCTTCCCCCTCC
GATTTACTTCCCCTCCCCCCCCCGCTTCCCTATCGCTGTCTCTCTTTCTTTCTTTTT
TTTATTGTCGTTGCAGCATCCATCTCTTTTCTCGTTTCAATCCATCTGCACGTCAAT
TTTTTTCTTTTTCTTTCTTTTTGATTTTCCCTTTCTTTTCTCCTGTTTCTGATCTCGAA
TCAGGACCTCTCCGGGACGGAGATCCTCCCGTTTCTATATCAACCCATTTTTCCC
GCCCAATTCCTCCCGTTACCATAGGGCTATGCAGCTTCTCCCTTGAATGAGCATTC
CATCCCAGTCAAAACCACACAACTACTATTGCACATCACTCGGCCGATTTACTGCT
TCGGTTGGTGAAGCAGGTATGCGGCCAATTGAAGTCGAGACTCCCCTATTTCCGT
TGGTTTTCGCATTCTTGTCCACGGAACGGCACCAGT
SEQ ID NO: 7 Synthetic constitutive promoter BBa_K3046007|PLEAPgfaA_1|medium: 
TTCACACCAGAGGCAAAATCTACTGTGGGATACTACTCAAGTACAAAGAGGGGCT
CCCTCACGATTTAGAGGTCTGTATCTGCGAGATTCTATGATTAAGGGTAGCAACAT
GAATAAGACGCATAGCAACTACCGACAGGGTGCTAATAACTTTATTTGTCGCCAAT
AACTTGATTTTTTGCATTTTCCACTTGTAGATGACTACATGCGTATCTATAAATAATA
GATAGGGGGGGAGAAGTTTCTACCTAGAACTAAAAATATTATTAGAAACCTTTGGG
TGTGTTATTTTTAGAAACTTTCGCCATCGTCTATACTCGAACAAAATCCATGGAGAC
AGGGAGAAATATTGACTGGTAGCTAATAATAGTCGGTATGTTATTTTTGACCTCCC
AAGTGGGGATGATTACCTCTTTAATTCTTAGTAGATATAGTTAGCACTCTATTTTCC
ATGCCATGGGATGGTCGGTTGTCCTGAACCGAGTCACGAGTGAGACATACCAATA
GACTGTGTGAGGCGCAGAGTGGGCTTATTGGCCACTTCCTCATTGGATGAATGAT
TGCGCCCTGGCGGTGGTCCATAGCAGCTTCCCTGGTCTGTGTCAAGGTGGGCCG
ATCTCACAACATAAAACATAACAGCTCCCCCTCCTTCCAACTGTTTGTTGATCTTAA
 
TCAACTTCCCCCTTTCTTTCAACTTCTCTCCTTCCTCCCCTCCTTCTCCCTCCCTCC
TCCAACTCTCTCCTTCACCAAATCTATCACATCCTCTTTCCTACTTTTTTTATGTTAT
CCTCCATAAATCTCTCATTTGTTGACATCCTATACCTTTCCTCACCCCACACTTACC
GTTTACGTTTCGTTCAGTATTCGTTCTCTTTCTTAGGCCACCTTCTCTTAGATCTCT
ACCCTTACACTTATCATAGAACTCTTAGTCTTTCCCTTCTCCTCCCTTAATCTCCTC
TTAACATTCATCAAC
SEQ ID NO: 8 Synthetic constitutive promoter BBa_K3046008|PLEAPhfbD_1|high:

ACTCTACGAGTAGGCTAGCATTCTAAGCCTCAGAGATTCAGTATCCGCGCCAGAG
CGGGACGGCCATCTGCAAGGGGTAAGACGCACCCCGCTGGGGTGGGAATGTAG
CGAACTTTTGTTATCATGTTCTCGCATAATGCCCTTGTTTAAAATGATTCGGTATGA
CTGACATAGAGCCTAGTAGTGAGGAAGTGTACTTGGCGAGACAAGGGTACTGGC
GAATCGGCAGGTAGCGATAGTTTCTGGGAATGACTATAGGTATGCAAAAGACTCG
CCAAACATGGACGTTTCCTGATTTCAGCGCATGTTATGACGCAATGCTCAAGCCG
ATTACGTTATGGAAGATGAGATGCCATGCTACATTGATTGTGATCAGTTTGGTGAA
ATCAATACCATTACAAGCAGCATGATGCGAGGCTTGATGTGGGTTCATGAAGATCT
TTGTCCTTTATCTTACGGTTCTTCAAAGGCATAATGCGGGATAGCAATGGACGGGT
CTTTGTTGGCCTGGAGTAGAGCGAGATTTGGGAGGTAGCAAAAGGTATCGTTTAC
GAATAGAGCTCTGTGGGTCATCCTGCATGAAGAACCATTCAGCTCAAGCAATTTC
GACCTAGTCCATTGTCTTAAAAGTCTCCGGGTTATCTTGAAGATGAGCCAATTGAC
AAAATACACTGTGCTAGCCGACGCAGTTCTGGGCTGCACCGAGTGACATGTTGCG
TGCTTTGTCCTCTACATAAAGCTTGCCATTGGTGAGAGGCCAGAGGGTCAATCAC
GTTACAGGATCAATATCTTGTCTCAAGTATGCAAATAGGACGAATAACTGATATAC
CAAGTCAAGTGGCTTATAAGCCCCAAATAGGACAGTCTCCATGGGCGAGAGAGCT
ATATAAACCATCAAAAACCCCTATCTTTCTACTGCATTTTTTCCTCATCACAACACA
ATTCCTGTATCTTATCATTTTCAAAAGCAATACTACACTTCTTCTCAGGCTTACAAA
GAACTCAATCTATCAC

## Claims

1. A computer-implemented method for generating a synthetic promoter and/or synthetic terminator for expressing a protein of interest in a cell, the method comprising:
i. obtaining gene sequences, such as functional sequences and/or coding sequences, of genomes of multiple closely related species;
ii. identifying at least one homolog group, each homolog group comprising multiple gene sequences that are homologs;
iii. identifying gene statistics for each gene sequence of the homolog groups, wherein the gene statistics are based on performance parameters and are a prediction of the strength of promoters or terminators of each respective gene sequence;
iv. identifying, for each homolog group, a set of functional DNA contexts comprising, for each gene sequence of respective homolog group:
∘ in the case of generation of a synthetic promoter: an upstream DNA sequence, said upstream DNA sequence being upstream of the start codon of the gene sequence, in the respective genome, and having a length greater than the average intergenic distance between divergently transcribed genes in said given genome; or
∘ in the case of generation of a synthetic terminator: a downstream DNA sequence, said downstream DNA sequence being downstream of the stop codon of the gene sequence, in the respective genome, and having a length greater than the average intergenic distance between divergently transcribed genes in said given genome;
v. building, for each homolog group, a multiple sequence alignment (MSA), each MSA consisting of the set of functional DNA contexts of said homolog group,
vi. iteratively fitting, for each MSA, a hidden markov model (HMM), wherein the fitting is repeated until convergence and/or until a predetermined number of iterations have been carried out, thereby obtaining a starting consensus sequence for each MSA;
vii. identifying multiple model features of the HMM, the model features being indicative of regions of high conservation in said starting consensus sequences, and generating a max-score matrix (FMS) comprising the maximum scores for each model feature for each HMM;
viii. forming a statistical model, such as PLSR, for modeling gene statistics as a function of FMS;
ix. identifying activity regulating features, by calculating a correlation coefficient between parameters of the statistical model and features scores of the FMS, and recognizing said activity regulating features as model features of which said correlation coefficient is above a predetermined threshold, such as 0.5,
x. optimizing, for each HMM, the starting consensus sequence, by, for each activity regulating feature, calculating the gene statistics upon inserting/replacing the activity regulating feature into the starting consensus sequence or a partially optimized consensus sequence, and
∘ where said insertion/replacement leads to an improvement in the gene statistics, such as an increase by a predetermined threshold, accepting said insertion/replacement;
∘ otherwise said insertion/replacement is discarded;
thereby generating a synthetic promoter and/or synthetic terminator for expressing a protein of interest in a cell.

2. The method according to claim 1, wherein the performance parameters are selected from the group consisting of: transcription rate, preferably based on tRNA adaptation index and/or number of effective codons; transcription profiling, preferably based on RNA sequencing data; and protein quantification, preferably measured by mass spectrometry; and/or
wherein the gene statistics is, for each gene sequence, a linear combination of the tRNA adaptation index and the number of effective codons.

3. The method according to any one of the preceding claims, wherein gene sequences of at least 10 closely related species are obtained; and/or
wherein the closely related species are of the same genus or related genera within a family; and/or
wherein the species are selected from any one of Aspergillus, Saccharomyces, Kluyveromyces, Komagataella, Barnettozyma, Cyberlindnera, Phaffomyces, Physcomitrium, Starmera, Wickerhamomyces, Yarrowia or Trichoderma.

4. The method according to any one of the preceding claims, wherein the homolog groups are identified such that each homolog groups contains at least 80% of the coding genes in any one of the closely related species, wherein the homolog groups are defined across multiple species.

5. The method according to any one of the preceding claims, wherein for each iteration of the HMM, the HMM is used to create an updated MSA that is used to fit an updated HMM.

6. The method according to any one of the preceding claims, wherein the fitting is repeated until convergence that is defined as no change of the consensus sequence, such as the most likely outcome of the HMM, for a predetermined number of iterations, such as five.

7. The method according to any one of the preceding claims, wherein the regions of high conservation are defined as regions of high information content in the match states of the model; and/or
wherein the regions of high information content in the match states of the model are identified by:
a. performing a running mean for a predetermined length in match states of the model, such as between 6 and 9 match states,
b. identifying regions with a value above a predetermined threshold, such as 1.5 bits.

8. The method according to any one of the preceding claims, wherein merged regions of high information content in the match states of the model are identified by merging regions of high information content in the match states of the model that are separated by at maximum a predetermined number of positions, such as 3.

9. The method according to any one of the preceding claims, wherein the model features are identified by summarizing the consensus sequence of the regions of high information content and/or the merged regions of high information content in the match states of the model.

10. The method according to any one of the preceding claims, wherein optimization of the starting consensus sequence is carried out from the activity regulating feature with the highest correlation to the parameters of the statistical model to the activity regulating feature with the lowest correlation to the parameters of the statistical model.

11. The method according to any one of the preceding claims, wherein the activity regulating feature is tested for insertion/replacement at the position of the FMS and 85% of the FMS of that activity regulating feature.

12. A statistical model for generation of synthetic promoters and/or terminators, the model generated according to the method of any one of claims 1-11.

13. A data processing system for generating or identifying synthetic promoters and/or synthetic terminators, the system comprising an input device, a central processing unit, a memory, and an output device, wherein said data processing system has stored therein data representing sequences of instructions which when executed cause the method of any one of claims 1-11 to be performed, the memory further comprising the statistical model for generation of synthetic promoters and/or synthetic terminators according to claim 12.

## Patentansprüche

1. Computerimplementiertes Verfahren zum Erzeugen eines synthetischen Promotors und/oder synthetischen Terminators zum Exprimieren eines Proteins von Interesse in einer Zelle, wobei das Verfahren Folgendes umfasst:
i. Erhalten von Gensequenzen, wie funktionellen Sequenzen und/oder codierenden Sequenzen, von Genomen mehrerer eng verwandter Arten;
ii. Identifizieren mindestens einer Homologengruppe, wobei jede Homologengruppen mehrere Gensequenzen umfasst, die Homologe sind;
iii. Identifizieren von Genstatistiken für jede Gensequenz der Homologengruppen, wobei die Genstatistiken auf Leistungsparametern basieren und eine Vorhersage der Stärke von Promotoren oder Terminatoren jeder jeweiligen Gensequenz sind;
iv. Identifizieren eines Satzes funktioneller DNA-Kontexte für jede Homologengruppe, die für jede Gensequenz der jeweiligen Homologengruppe Folgendes umfassen:
∘ in dem Fall einer Erzeugung eines synthetischen Promotors: eine stromaufwärts gelegene DNA-Sequenz, wobei die stromaufwärts gelegene DNA-Sequenz stromaufwärts des Startcodons der Gensequenz in dem jeweiligen Genom ist und eine Länge aufweist, die größer als der durchschnittliche intergene Abstand zwischen divergent transkribierten Genen in dem gegebenen Genom ist; oder
∘ in dem Fall einer Erzeugung eines synthetischen Terminators: eine stromabwärts gelegene DNA-Sequenz, wobei die stromabwärts gelegene DNA-Sequenz stromabwärts des Stoppcodons der Gensequenz in dem jeweiligen Genom ist und eine Länge aufweist, die größer als der durchschnittliche intergene Abstand zwischen divergent transkribierten Genen in dem gegebenen Genom ist;
v. Erstellen eines Mehrfachsequenzalignments (multiple sequence alignment, MSA) für jede Homologengruppe, wobei jedes MSA aus dem Satz funktioneller DNA-Kontexte der Homologengruppe besteht,
vi. iteratives Anpassen eines verborgenen Markow-Modells (hidden markov model, HMM) für jedes MSA, wobei das Anpassen bis zu einer Konvergenz und/oder bis eine vorbestimmte Anzahl von Iterationen vorgenommen worden ist, wiederholt wird, wodurch eine Consensus-Startsequenz für jedes MSA erhalten wird;
vii. Identifizieren mehrerer Modellmerkmale des HMM, wobei die Modellmerkmale Bereiche hoher Erhaltung in den Consensus-Startsequenzen anzeigen, und Erzeugen einer Max-Score-Matrix (FMS), umfassend die maximalen Scores für jedes Modellmerkmal für jedes HMM;
viii. Ausbilden eines statistischen Modells, wie PLSR, zum Modellieren von Genstatistiken als eine Funktion des FMS;
ix. Identifizieren von aktivitätsregulierenden Merkmalen durch Berechnen eines Korrelationskoeffizienten zwischen Parametern des statistischen Modells und Merkmalsscores des FMS und Erkennen der aktivitätsregulierenden Merkmale als Modellmerkmale, von denen der Korrelationskoeffizient über einem vorbestimmten Schwellenwert, wie 0,5, ist,
x. Optimieren der Consensus-Startsequenz für jedes HMM durch Berechnen der Genstatistiken beim Einfügen/Ersetzen des aktivitätsregulierenden Merkmals in die Consensus-Startsequenz oder eine teilweise optimierte Consensus-Sequenz für jedes aktivitätsregulierende Merkmal und
∘ wobei die Einfügung/Ersetzung zu einer Verbesserung der Genstatistiken führt, wie einer Erhöhung um einen vorbestimmten Schwellenwert, der die Einfügung/Ersetzung akzeptiert;
∘ die Einfügung/Ersetzung andernfalls verworfen wird; wodurch ein synthetischer Promotor und/oder synthetischer Terminator zum Exprimieren eines Proteins von Interesse in einer Zelle erzeugt wird.

2. Verfahren nach Anspruch 1, wobei die Leistungsparameter ausgewählt sind aus der Gruppe bestehend aus:
Transkriptionsrate, vorzugsweise basierend auf einem tRNA-Anpassungsindex und/oder einer Anzahl effektiver Codons;
Transkriptionsprofilieren, vorzugsweise basierend auf RNA-Sequenzierungsdaten; und Proteinquantifizierung, vorzugsweise durch Massenspektrometrie gemessen; und/oder
wobei die Genstatistiken für jede Gensequenz eine lineare Kombination des tRNA-Anpassungsindexes und der Anzahl effektiver Codons sind.

3. Verfahren nach einem der vorhergehenden Ansprüche, wobei Gensequenzen von mindestens 10 eng verwandten Arten erhalten werden; und/oder
wobei die eng verwandten Arten von der gleichen Gattung oder verwandten Gattungen innerhalb einer Familie sind; und/oder
wobei die Arten ausgewählt sind aus Aspergillus, Saccharomyces, Kluyveromyces, Komagataella, Barnettozyma, Cyberlindnera, Phaffomyces, Physcomitrium, Starmera, Wickerhamomyces, Yarrowia oder Trichoderma.

4. Verfahren nach einem der vorhergehenden Ansprüche, wobei die Homologengruppen derart identifiziert werden, dass jede Homologengruppe mindestens 80 % der codierenden Gene in einer der eng verwandten Arten enthält, wobei die Homologengruppen über mehrere Arten hinweg definiert sind.

5. Verfahren nach einem der vorhergehenden Ansprüche, wobei für jede Iteration des HMM das HMM verwendet wird, um ein aktualisiertes MSA zu kreieren, das verwendet wird, um ein aktualisiertes HMM anzupassen.

6. Verfahren nach einem der vorhergehenden Ansprüche, wobei das Anpassen bis zu einer Konvergenz wiederholt wird, die als keine Änderung der Consensus-Sequenz, wie des wahrscheinlichsten Ergebnisses des HMM, für eine vorbestimmte Anzahl von Iterationen, wie fünf, definiert ist.

7. Verfahren nach einem der vorhergehenden Ansprüche, wobei die Bereiche hoher Erhaltung als Bereiche hohen Informationsgehalts in den Übereinstimmungszuständen des Modells definiert sind; und/oder
wobei die Bereiche hohen Informationsgehalts in den Übereinstimmungszuständen des Modells durch Folgendes identifiziert werden:
a. Durchführen eines gleitenden Mittelwerts für eine vorbestimmte Länge in Übereinstimmungszuständen des Modells, wie zwischen 6 und 9 Übereinstimmungszuständen,
b. Identifizieren von Bereichen mit einem Wert über einem vorbestimmten Schwellenwert, wie 1,5 Bits.

8. Verfahren nach einem der vorhergehenden Ansprüche, wobei zusammengeführte Bereiche hohen Informationsgehalts in den Übereinstimmungszuständen des Modells durch Zusammenführen von Bereichen hohen Informationsgehalts in den Übereinstimmungszuständen des Modells identifiziert werden, die durch maximal eine vorbestimmte Anzahl von Positionen, wie 3, getrennt sind.

9. Verfahren nach einem der vorhergehenden Ansprüche, wobei die Modellmerkmale durch Zusammenfassen der Consensus-Sequenz der Bereiche hohen Informationsgehalts und/oder der zusammengeführten Bereiche hohen Informationsgehalts in den Übereinstimmungszuständen des Modells identifiziert werden.

10. Verfahren nach einem der vorhergehenden Ansprüche, wobei eine Optimierung der Consensus-Startsequenz von dem aktivitätsregulierenden Merkmal mit der höchsten Korrelation zu den Parametern des statistischen Modells bis zu dem aktivitätsregulierenden Merkmal mit der niedrigsten Korrelation zu den Parametern des statistischen Modells vorgenommen wird.

11. Verfahren nach einem der vorhergehenden Ansprüche, wobei das aktivitätsregulierende Merkmal auf eine Einfügung/Ersetzung an der Position des FMS und 85 % des FMS dieses aktivitätsregulierenden Merkmals geprüft wird.

12. Statistisches Modell zur Erzeugung synthetischer Promotoren und/oder Terminatoren, wobei das Modell gemäß dem Verfahren nach einem der Ansprüche 1 bis 11 erzeugt wird.

13. Datenverarbeitungssystem zum Erzeugen oder Identifizieren synthetischer Promotoren und/oder synthetischer Terminatoren, wobei das System eine Eingabevorrichtung, eine zentrale Verarbeitungseinheit, einen Speicher und eine Ausgabevorrichtung umfasst, wobei das Datenverarbeitungssystem darin gespeicherte Daten aufweist, die Sequenzen von Anweisungen darstellen, die, wenn sie ausgeführt werden, bewirken, dass das Verfahren nach einem der Ansprüche 1 bis 11 durchgeführt wird, wobei der Speicher ferner das statistische Modell zur Erzeugung synthetischer Promotoren und/oder synthetischer Terminatoren nach Anspruch 12 umfasst.

## Revendications

1. Procédé mis en œuvre par ordinateur permettant la génération d'un promoteur synthétique et/ou d'un facteur de terminaison synthétique pour l'expression d'une protéine d'intérêt dans une cellule, le procédé comprenant :
i. l'obtention de séquences géniques, telles que des séquences fonctionnelles et/ou des séquences codantes, de génomes de multiples espèces étroitement apparentées ;
ii. l'identification d'au moins un groupe d'homologues, chaque groupe d'homologues comprenant de multiple séquences géniques qui sont homologues ;
iii. l'identification de statistiques géniques pour chaque séquence génique des groupes d'homologues, dans lequel les statistiques géniques sont basées sur des paramètres de performance et constituent une prédiction de la force de promoteurs ou de facteurs de terminaison de chaque séquence génique respective ;
iv. l'identification, pour chaque groupe d'homologues, d'un ensemble de contextes d'ADN fonctionnels comprenant, pour chaque séquence génique de groupe d'homologues respectif :
∘ dans le cas de la génération d'un promoteur synthétique : une séquence d'ADN en amont, ladite séquence d'ADN en amont étant située en amont du codon d'initiation de la séquence génique, dans le génome respectif, et ayant une longueur supérieure à la distance intergénique moyenne entre des gènes transcrits de manière divergente dans ledit génome ; ou
∘ dans le cas de la génération d'un facteur de terminaison synthétique : une séquence d'ADN en aval, ladite séquence d'ADN en aval étant en aval du codon d'arrêt de la séquence génique, dans le génome respectif, et ayant une longueur supérieure à la distance intergénique moyenne entre des gènes transcrits de manière divergente dans ledit génome donné ;
v. la construction, pour chaque groupe d'homologues, d'un alignement multiple de séquences (MSA), chaque MSA consistant en l'ensemble des contextes d'ADN fonctionnels dudit groupe d'homologues,
vi. l'ajustement itératif, pour chaque MSA, d'un modèle de Markov caché (HMM), dans lequel l'ajustement est répété jusqu'à convergence et/ou jusqu'à ce qu'un nombre prédéterminé d'itérations ait été effectué, obtenant ainsi une séquence consensus initiale pour chaque MSA ;
vii. l'identification de plusieurs caractéristiques de modèle du HMM, les caractéristiques de modèle étant indicatives de régions de forte conservation dans lesdites séquences consensus initiales, et la génération d'une matrice des scores maximaux (FMS) comprenant les scores maximaux pour chaque caractéristique de modèle pour chaque HMM ;
viii. la formation d'un modèle statistique, tel que la PLSR, pour modéliser des statistiques géniques en fonction de FMS ;
ix. l'identification de caractéristiques de régulation d'activité, en calculant un coefficient de corrélation entre des paramètres du modèle statistique et des scores de caractéristiques de la FMS, et en reconnaissant lesdites caractéristiques de régulation d'activité comme des caractéristiques de modèle dont ledit coefficient de corrélation est supérieur à un seuil prédéterminé, par exemple 0,5,
x. l'optimisation, pour chaque HMM, de la séquence consensus initiale, en calculant, pour chaque caractéristique de régulation d'activité, les statistiques géniques lors de l'insertion/substitution de la caractéristique de régulation d'activité dans la séquence consensus initiale ou dans une séquence consensus partiellement optimisée, et
∘ lorsque ladite insertion/substitution conduit à une amélioration des statistiques géniques, telle qu'une augmentation d'un seuil prédéterminé, l'acceptation de ladite insertion/substitution ;
∘ sinon ladite insertion/substitution est rejetée ; générant ainsi un promoteur synthétique et/ou un facteur de terminaison synthétique pour exprimer une protéine d'intérêt dans une cellule.

2. Procédé selon la revendication 1, dans lequel les paramètres de performance sont choisis dans le groupe constitué de : taux de transcription, de préférence basé sur un indice d'adaptation d'ARNt et/ou un nombre de codons effectifs ; profilage de transcription, de préférence basé sur des données de séquençage d'ARN ; et quantification des protéines, de préférence mesurée par spectrométrie de masse ; et/ou
dans lequel les statistiques géniques sont, pour chaque séquence génique, une combinaison linéaire de l'indice d'adaptation d'ARNt et du nombre de codons effectifs.

3. Procédé selon l'une quelconque des revendications précédentes, dans lequel des séquences géniques d'au moins 10 espèces étroitement apparentées sont obtenues ; et/ou
dans lequel les espèces étroitement apparentées appartiennent au même genre ou à des genres apparentés au sein d'une même famille ; et/ou
dans lequel les espèces sont choisies parmi l'un quelconque parmi Aspergillus, Saccharomyces, Kluyveromyces, Komagataella, Barnettozyma, Cyberlindnera, Phaffomyces, Physcomitrium, Starmera, Wickerhamomyces, Yarrowia ou Trichoderma.

4. Procédé selon l'une quelconque des revendications précédentes, dans lequel les groupes d'homologues sont identifiés de sorte que chaque groupe d'homologues contienne au moins 80 % des gènes codants dans l'une quelconque des espèces étroitement apparentées, dans lequel les groupes d'homologues sont définis à travers de multiples espèces.

5. Procédé selon l'une quelconque des revendications précédentes, dans lequel, pour chaque itération du HMM, le HMM est utilisé pour créer un MSA mis à jour qui est utilisé pour ajuster un HMM mis à jour.

6. Procédé selon l'une quelconque des revendications précédentes, dans lequel l'ajustement est répété jusqu'à convergence, définie comme l'absence de changement de la séquence consensus, par exemple le résultat le plus probable du HMM, pour un nombre prédéterminé d'itérations, par exemple cinq.

7. Procédé selon l'une quelconque des revendications précédentes, dans lequel les régions de forte conservation sont définies comme des régions à contenu informationnel élevé dans les états de correspondance du modèle ; et/ou
dans lequel les régions à contenu informationnel élevé dans les états de correspondance du modèle sont identifiées par :
a. la réalisation d'une moyenne mobile sur une longueur prédéterminée dans des états de correspondance du modèle, par exemple entre 6 et 9 états de correspondance,
b. l'identification de régions présentant une valeur supérieure à un seuil prédéterminé, par exemple 1,5 bits.

8. Procédé selon l'une quelconque des revendications précédentes, dans lequel des régions fusionnées à contenu informationnel élevé dans les états de correspondance du modèle sont identifiées en fusionnant des régions à contenu informationnel élevé dans les états de correspondance du modèle qui sont séparées par au maximum un nombre prédéterminé de positions, par exemple 3.

9. Procédé selon l'une quelconque des revendications précédentes, dans lequel les caractéristiques de modèle sont identifiées en résumant la séquence consensus des régions à contenu informationnel élevé et/ou des régions fusionnées à contenu informationnel élevé dans les états de correspondance du modèle.

10. Procédé selon l'une quelconque des revendications précédentes, dans lequel l'optimisation de la séquence consensus initiale est effectuée de la caractéristique de régulation d'activité présentant la corrélation la plus élevée avec les paramètres du modèle statistique à la caractéristique de régulation d'activité présentant la corrélation la plus faible avec les paramètres du modèle statistique.

11. Procédé selon l'une quelconque des revendications précédentes, dans lequel la caractéristique de régulation d'activité est testée pour insertion/substitution à la position de la FMS et à 85 % de la FMS de cette caractéristique de régulation d'activité.

12. Modèle statistique permettant la génération de promoteurs et/ou de facteurs de terminaison synthétiques, le modèle étant généré selon le procédé selon l'une quelconque des revendications 1 à 11.

13. Système de traitement de données permettant la génération ou l'identification de promoteurs synthétiques et/ou de facteurs de terminaison synthétiques, le système comprenant un dispositif d'entrée, une unité centrale de traitement, une mémoire et un dispositif de sortie, dans lequel ledit système de traitement de données contient stockées dans celui-ci des données représentant des séquences d'instructions qui, lorsqu'elles sont exécutées, provoquent la mise en œuvre du procédé selon l'une quelconque des revendications 1 à 11, la mémoire comprenant également le modèle statistique permettant la génération de promoteurs synthétiques et/ou de facteurs de terminaison synthétiques selon la revendication 12.
